# FASCICULE DE BREVET EUROPEEN

(11) **EP 0 948 650 B1**
(45) Date de publication et mention de la délivrance du brevet: **06.07.2005**
(21) Numéro de dépôt: 97952971.6
(22) Date de dépôt: 22.12.1997
(51) Int. Cl.: C12Q 1/68, C12N 15/10

(54) **PROCEDE DESTINE A L'ELIMINATION DE SEQUENCES SPECIFIQUES LORS DE LA PRODUCTION DE BANQUES D'ADN**
VERFAHREN ZUR ELIMINIERUNG VON SPEZIFISCHEN SEQUENZEN WÄHREND DER HERSTELLUNG VON DNS-BIBLIOTHEKEN
METHOD FOR ELIMINATING SPECIFIC SEQUENCES WHEN CONSTRUCTING DNA LIBRARIES

(30) Priorité: 23.12.1996 FR 9615854
(43) Date de publication de la demande: 13.10.1999
(73) Titulaire: Serono Genetics Institute S.A., 91030 Evry Cedex (FR)
(72) Inventeur: NAHAS, Nasri, F-75015 Paris (FR); DUMAS MILNE EDWARDS, Jean-Baptiste, F-75006 Paris (FR)
(74) Mandataire: Ahner, Francis
(86) Numéro de dépôt international: PCT/FR1997/002378
(87) Numéro de publication internationale: WO 1998/028439

(56) Documents cités:
- WO-A-87/01730
- WO-A-88/07585
- WO-A-92/08791
- HAKVOORT T B M ET AL: "PREPARATION OF A DIFFERENTIALLY EXPRESSED, FULL-LENGTH CDNA EXPRESSION LIBRARY BY RECA-MEDIATED TRIPLE-STRAND FORMATION WITH SUBTRACTIVELY ENRICHED CDNA FRAGMENTS" NUCLEIC ACIDS RESEARCH, vol. 24, no. 17, 1 septembre 1996, OXFORD GB, pages 3478-3480, XP000625347
- BONALDO M F ET AL: "NORMALIZATION AND SUBTRACTION: TWO APPROACHES TO FACILITATE GENE DISCOVERY" GENOME RESEARCH, vol. 6, no. 9, septembre 1996, ING HARBOR LABORATORY PRESS US, pages 791-806, XP002039972
- KOOB M ET AL.: "RECA-AC: SINGLE-SITE CLEAVAGE OF PLASMIDS AND CHROMOSOMES AT ANY PREDETERMINED RESTRICTION SITE" NUCLEIC ACIDS RESEARCH, vol. 20, no. 21, 1992, OXFORD GB, pages 5831-5836, XP002039973 cité dans la demande
- VESELKOV A G ET AL: "A NEW CLASS OF GENOME RARE CUTTERS" NUCLEIC ACIDS RESEARCH, vol. 24, no. 13, 1996, OXFORD GB, pages 2483-2487, XP002022889

## Description

La présente invention concerne un procédé destiné notamment à éliminer des séquences spécifiques de banques d'ADN.

La présente invention concerne plus particulièrement un procédé destiné à éliminer au moins une séquence spécifique, notamment abondante, d'une banque d'ADNc.

Le séquençage aléatoire de clones d'ADNc est, depuis plusieurs années, utilisé comme moyen d'accéder à des profils d'expression différentiels ainsi qu'à la découverte de nouvelles séquences Typiquement, il s'agit d'extraire les ARNs messagers (ARNm) de cellules ou tissus particuliers, de cloner les ADNc correspondants, puis de sélectionner au hasard des clones qui seront séquencés. Si l'objectif est la recherche de séquences nouvelles, l'opération de séquençage exhaustif d'une banque d'ADNc est rendue coûteuse à cause de la redondance de la population d'ARNm En effet, si, selon les tissus, 15 000 à 50 000 espèces moléculaires d'ARNm sont exprimées, celles-ci représentent un total de 200 à 500 000 molécules dans les cellules (Davidson et Britten, 1979)

Dans les cellules, les messagers sont exprimés à des taux différents selon les espèces moléculaires. Ainsi, 10 a 20 espèces moléculaires peuvent représenter 10 à 20 % de molécules d'ARNm De nombreux auteurs ont étudié la redondance des banques et montré que, dans certains cas, une seule espèce moléculaire pouvait compter pour plus de 20 % des clones Afin de préciser ces données, les inventeurs ont étudié dans plusieurs tissus humains la redondance des séquences d'ARNm Pour cela, ils ont classé par groupes de similarité les séquences de clones sélectionnés au hasard au sein de banques d'extrémités 5'

Pour 2 à 3 000 clones sequencés, les fractions de clones appartenant à des groupes de similarité contenant 10 séquences ou plus sont respectivement de 39 %, 6,8 %, 22 %, 54 %, 34 %, 46 %, 27 %, 27 %, 7 %, 39 %, pour des tissus ou des cellules comme le placenta, le cerveau, la rate, le pancréas, le colon, les lymphocytes, le foie, le rein, l'ovaire, le coeur (tableau 1)

**TABLEAU I**

| TISSUS | % TOP15 | % TOP20 | % TOP30 |
|---|---|---|---|
| Pancréas | 46 | 48 | 52 |
| Rein | 15 | 17 | 20 |
| Colon | 18 | 21 | 26 |
| Lymphocyte | 25 | 28 | 33 |
| Rein | 23 | 26 | 30,5 |
| Muscle Dystrophique | 26 | 27,5 | 31,5 |
| Foie | 27 | 29 | 33 |
| Ovaire | 7,5 | 8 | 10 |
| Cerveau | 8 | 9 | 11 |

Tableau I Profil d'abondance des clones les plus représentés des banques d'ADNc étiquetées. Les banques sont construites à partir d'ARNs messagers de pancréas, rate, colon, lymphocyte, rein, muscle dystrophique, foie, ovaire, et cerveau Le tableau montre la distribution des 15, 20 ou 30 clones les plus représentés dans chacune de nos banques.

Ainsi, le coût d'une séquence nouvelle peut être très important , en effet, très rapidement au cours du séquençage, entre le quart et la moitié des séquences ont déjà été réalisées Pour chaque séquence, il y a donc entre 50 et 75 % de chance que cette séquence soit déjà faite. Pour limiter le coût de nouvelles séquences, plusieurs solutions ont été proposées

La première consiste à normaliser les banques d'ADNc ou la population d'ARNm de départ Cela consiste à diminuer la fréquence des séquences abondantes de telle sorte qu'elle avoisine la fréquence des séquences rares Plusieurs méthodes de normalisation ont été proposées L'une d'elles consiste à utiliser l'ADN génomique comme matrice de normalisation (Weisman, 1987) Dans ce cas, la distnbution des clones d' ADNc obtenus devrait être corréléc avec le nombre d'exons du gène

La méthode de normalisation la plus employée est basée sur la cinétique d'hybridation Lorsqu'une population d'acides nucléiques est dénaturée, la vitesse de renaturation (ou de réappariement des molécules) est proportionnelle au nombre d'espèces moléculaires présentes. Autrement dit, plus une espèce moléculaire sera présente dans la population d'ADNc, plus les molécules d'ADNc correspondantes seront rapidement renaturées (Britten et al., 1974 , Young & Anderson, 1985). La principale difficulté technique de cette approche est de séparer correctement les populations de molécules appariées des populations de molécules non appariées

La plupart des méthodes font appel à une chromatographie sur résine d'hydroxyapatite qui libère les molécules simple brin et les molécules double brin à des concentrations différentes d'ions phosphate (Patanjali et al., 1991) Des stratégies faisant appel à des systèmes semi-solides pour éliminer des duplex ADN-ADN ou des hétéroduplex ADN-ARN ont été récemment proposées (Sasaki et al, 1994) Dans ce cas, des ADNc sont synthétisés à l'aide d'amorces auxquelles des billes de latex ont été accrochées à l'extrémité 5' Les populations d'ADNc obtenues sont utilisées comme matrice d'hybridation avec une population d'ARNm et les hétéroduplex sont collectés par centrifugation.

Toutes les techniques de normalisation par hybridation ont la limitation de ne pas permettre la discrimination entre les séquences faiblement divergentes. Récemment, Bento Soares (Soares et al , 1994 , Soares & Efstratiadis, 1995) a proposé une méthode permettant de soustraire les séquences abondantes des banques d'ADNc Cette méthode est basée sur la production en masse de plasmides simple brin d'une banque d'ADNc produite par amorçage ancré sur la queue poly-A des ARNm L'extrémité définie ainsi obtenue est utilisée pour amorcer la synthèse de segments courts d'ADN sur la matrice plasmidique simple brin. Ceci permet d'obtenir des molécules localement bicaténaires. La faible taille du fragment néosynthétisé (200 ± 20-nt) est un atout dans la stratégie de soustraction car les séquences non traduites en 3' sont beaucoup plus divergentes que les séquences codantes. De plus, comme tous les fragments ont à peu près la même taille, la cinétique d'hybridation pour l'ensemble de la population plasmidique est plus simple. Par la suite, la normalisation de la banque est réalisée en dénaturant les duplex et en tirant parti de leurs cinétiques de différentes réhybridations La séparation des plasmides circulaires simple brin et des hétéroduplex se fait alors au moyen de colonnes d'hydroxyapatite Puis les plasmides simple brin sont transformés en double brin par extension d'amorces et transfectés dans des bactéries. Cette méthode présente l'avantage de placer les partenaires d'hybridation en condition équimolaire , par contre, elle a deux inconvénients
1) la purification des clones non recombinants,
2) pour une bonne hybridation, la taille du fragment néosynthétisé à partir des plasmides circulaires simple brin doit être approximativement de 200 nt.

Dans le cas des banques d'extrémités 5' produites ici, la taille moyenne des insertions est de 200 à 250 nt. Ainsi, la stratégie de normalisation de Soares est difficilement applicable.

La deuxième solution proposée pour limiter le coût de nouvelles séquences consiste à ordonnancer la banque d'ADNc sur des filtres et à hybrider les filtres avec des sondes correspondant aux messagers les plus abondants puis à ordonner en plaque de microtitration les clones qui ne sont pas identifiés par la sonde Le choix des groupes de séquences les plus abondantes peut être fait après analyse de quelques milliers de clones (Lanfranchi et al , 1985), ou bien en utilisant des sondes constituées de l'ADNc total, du génome mitochondrial et/ou de quelques clones spécifiques choisis pour leur grande abondance (Adams et al, 1995) Cette approche peut être séduisante car, contrairement à la normalisation, elle permet le repérage puis l'élimination, grâce à la sélection de clones non reconnus, des clones correspondant aux séquences les plus abondantes.

Toutefois, les manipulations d'ordonnancement des clones sur filtre et d'hybridation sont lourdes et la sélection des clones non reconnus par les sondes utilisées est souvent délicate (bruit de fond d'hybridation, identification précise des clones, erreurs possibles lors du réordonnancement ou du repiquage de la banque).

Dans la présente invention, l'élimination est réalisée en rendant inclonables les plasmides ayant des insertions correspondant aux séquences spécifiques ciblées parmi les plus abondantes. Cela est effectué en dirigeant sélectivement l'activité d'enzymes de restriction sur les séquences à éliminer Les clones ainsi linéarisés ne sont plus capables de transformer des bactéries.

C'est pourquoi, la présente invention concerne un procédé destiné à éliminer spécifiquement au moins un type de plasmide d'ADN double brin dans un ensemble de plasmides notamment dans le cas de la création d'une banque, caractérisé en ce que.
- on prépare des fragments d'acides nucléiques capables de former des structures en triplex et dont la séquence correspond à une séquence du plasmide, plus particulièrement à un fragment de la séquence du groupe de clones à éliminer et comportant, en outre, un site de reconnaissance pour une enzyme de restriction sensible à la méthylation ,
- on mélange l'ensemble des plasmides avec lesdits fragments d'acides nucléiques pour former des triplex sur les plasmides à éliminer ;
- on méthyle le mélange d'ADN obtenu ;
- on libère les triplex ;
- on digère le mélange obtenu avec l'enzyme de restriction sensible à la méthylation ;
- si nécessaire, on élimine ou on sépare les ADNs double brin linéaires correspondant aux plasmides à éliminer.

Dans un premier mode de réalisation, la formation du triplex se fera par hybridation d'un filament présynaptique avec la séquence cible, en présence d'une protéine à activité RecA

Ce procédé dérive de la stratégie dite du "Talon d'Achille" décrite par Koob et al (1992), mise en oeuvre pour des séquences d'ADN linéaires.

Dans ce procédé, la fixation d'une protéine, notamment la protéine RecA, sur une molécule d'ADN simple brin appropriée, catalyse la formation d'un triplex entre cet ADN simple brin (oligonucléotide) et un ADN double brin correspondant , le triplex ainsi formé protège de la méthylation les séquences qui y sont engagées

Lorsque les triplex sont libérés, les séquences correspondantes, non méthylées, deviennent les seules qui soient sensibles aux enzymes de restriction spécifiques choisies. On obtient ainsi dans le mélange des plasmides circulaires et dés ADNs linéaires correspondant aux plasmides que l'on souhaite éliminer, ce qui peut être fait de différentes façons comme cela sera explicité ci-après.

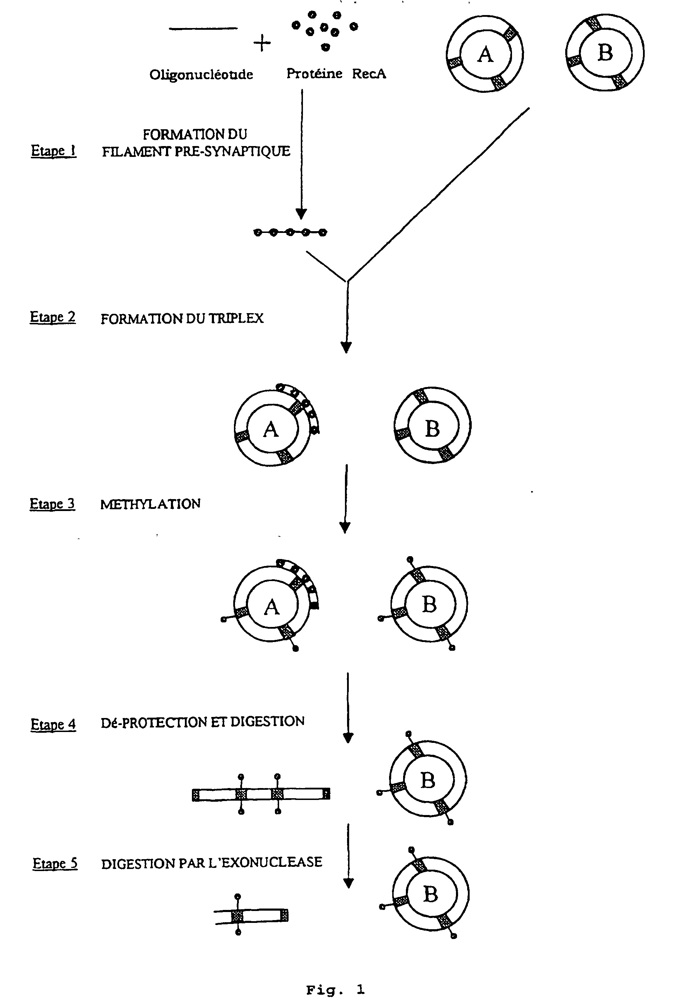

De façon plus particulière, la représentation schématique de l'invention appliquée à de l'ADN double brin plasmidique circulaire d'une banque d'ADNc est représentée sur la figure 1.

Le procédé selon la présente invention est plus particulièrement utilisable lorsque l'ensemble des plasmides de départ constitue une banque d'ADNc, comme cela a été mentionné précédemment

Le procédé est notamment utilisable pour l'élimination d'un clone portant une séquence spécifique connue

Dans la première étape, c'est-à-dire la formation du filament présynaptique, il est possible d'utiliser la protéine RecA qui est connue pour se fixer sur l'ADN simple brin (oligonucléotide) et former ledit filament présynaptique, mais il est également possible d'utiliser d'autres protéines, notamment une protéine dénommée "à activité RecA" qui sera définie ci-après comme étant capable de participer à la formation d'un filament présynaptique qui protègera de la méthylation la séquence d'ADN double brin ciblée, par formation d'un triplex avec celle-ci

Cette première étape de formation du filament présynaptique nécessite l'hydrolyse de l'ATP ou de son homologue modifié ATP(γS)

Bien entendu, la sélection de l'ADN simple brin employé pour former le filament présynaptique (oligonucléotide) constitue l'un des éléments clés de la présente invention puisqu'il sera nécessaire de choisir la séquence de cet oligonucléotide à partir de la (des) séquence(s) du (des) clones que l'on souhaite éliminer, et que cette séquence doit contenir un site de reconnaissance pour une enzyme de restriction "sensible à la méthylation" On entend par "enzyme de restriction sensible à la méthylation" une enzyme de restriction qui reconnaît son site lorsque celui-ci n'est pas méthylé Parmi les enzymes qui peuvent être utilisées, il faut citer notamment les enzymes citées dans le tableau II et plus particulièrement les enzymes HaeIII et MspI

Les oligonucléotides utilisés pour la formation du filament présynaptique seront de préférence des oligonucléotides comportant environ 30 nucléotides

**TABLEAU II**

| LISTE DES ENZYMES UTILISABLES DANS LA PROCEDURE D'ELIMINATION D'UN OU PLUSIEURS CLONES SPECIFIQUES NOTAMMENT ABONDANTS, D'UNE BANQUE D'ADN (liste non limitative) |
|---|
| **I. Méthylases** |
| M BamHI |
| M EcoRI |
| . M.pstI |
| M.ClaI |
| . M HaeIII |
| M MspI |
| M HhaI |
| M.HpaI |
| CpG Méthylase (Méthyle les residus C des dinucléotides (5' CG 3' Parmi les enzymes de restriction citées ci-dessous, cette méthylase affecte les suivantes AciI, BstUI, ClaI, HhaI, HinPI, et MspI) |

| **II. Enzymes de restriction à 6 bases** |
|---|
| . BamHI |
| . EcoRI |
| . PstI |
| . ClaI |

| **III. Enzymes de restriction à 4 bases** |
|---|
| . HaeIII |
| . MspI |
| . HhaI |
| HpaI |
| AciI |
| BstUI |
| . HinPI |

Lors de la deuxième étape, le filament présynaptique ainsi formé est mélangé avec l'ADN double brin, en l'occurrence les plasmides dans lesquels ont été clonées les séquences de la banque, dans des conditions qui permettent de former un tnplex stable d'ADN, ceci en présence d'ATP(γS) La réaction est également effectuée en présence d'un cation divalent, en particulier Mg2++.

La protéine RecA altère la topologie de la double hélice d'ADN et rend possible l'accès du filament présynaptique, dans le cas présent de l'ogligonucléotide, à sa séquence complémentaire sur l'ADN double brin des clones à éliminer Le complexe est stabilisé lors de cette étape

Il faut remarquer qu'il n'y a pas d'échange de brin possible entre le filament présynaptique et l'ADN double brin. En effet, un tel événement nécessiterait une forte efficacité d'hydrolyse qui ne peut être réalisée du fait qu'on utilise l'ATP(γS) dont l'efficacité d'hydrolyse est 100 fois inférieure à celle de l'ATP. Dans ces conditions, le triplex d'ADN reste stable et peut être conservé plusieurs jours à - 20°C.

Dans un second mode de réalisation, on formera les tnplex sur les zones à protéger de la méthylation, par hybridation des séquences cibles avec des PNAs, "Peptide Nucleic Acids" (Veselkov et al , 1996)

La troisième étape consiste a méthyler le mélange d'ADN constitué de molécules d'ADN double brin comportant localement, pour certains, des régions impliquées dans des triplex

Pour ce faire on utilise des méthyltransférases afin de méthyler spécifiquement les sites de reconnaissance des enzymes de restriction qui ont été choisies

Cette étape est bien entendu importante car elle protège de la coupure par les enzymes de restriction d'autres sites éventuellement présents ailleurs dans les insertions de la banque et dans le vecteur de clonage

Les régions spécifiques qui correspondent aux clones que l'on souhaite éliminer sont en principe protégées de la méthylation par la formation du triplex. Ces régions ne seront donc pas méthylées et seront ultérieurement les seules reconnues par l'enzyme de restriction correspondante.

Dans l'étape suivante, on libère les triplex, soit en dénaturant la protéine à activité RecA, notamment RecA, soit en dénaturant les triplex formés avec les PNA On obtient ainsi un mélange de plasmides circulaires double brin A la fin de cette étape, certains des plasmides comportent des régions qui ne sont pas méthylées car elles correspondent aux régions protégées par le triplex.

Dans la quatrième étape, on digère le mélange obtenu avec l'enzyme de restriction sensible à la méthylation, il est bien entendu qu'il peut s'agir d'une ou plusieurs enzymes de restriction, en fonction des sites qui ont été retenus pour être protégés par la formation des triplex

Cette digestion conduit à la formation d'ADN double brin linéaire à partir de tous les plasmides qui comportaient des sites protégés de la méthylation. Au contraire, les autres plasmides demeurent intacts.

Les enzymes de restriction utilisées doivent présenter des sites suffisamment fréquents pour que la probabilité de les trouver dans les insertions ciblées, dont la longueur varie de 200 a 250 nt, soit forte , c'est pourquoi on utilisera de préférence des enzymes de restriction dont la séquence de reconnaissance comporte 4 nucléotides

En outre, si l'on doit utiliser plusieurs enzymes, il est préférable que celles-ci soient actives dans les mêmes conditions, notamment de tampon , ceci évitera d'avoir à effectuer une digestion en plusieurs étapes, bien que ceci ne soit pas exclu dans le cadre de la présente invention.

Comme on l'a indiqué précédemment, deux enzymes ont été utilisées dans les exemples qui vont suivre , il s'agit de MspI et HaeIII

La dernière étape, élimination ou séparation des molécules d'ADN double brin linéaires, est facultative Il est possible dans certains cas d'utiliser des procédures permettant la séparation des plasmides double brin circulaires des fragments d'ADN double brin linéaires ; certaines de ces procédures ont été mentionnées précédemment. Mais, il est possible également d'éliminer par digestion les ADNs double brin linéaires, en particulier en les hydrolysant par des exonucléases, notamment l'exonucléase III qui hydrolyse l'ADN selon la direction 5'-3'.

Cette étape de digestion a l'avantage d'éviter d'éventuels réarrangements quand l'ADN linéaire est transformé dans les bactéries.

Cette étape est plus particulièrement intéressante pour diminuer la redondance de banques d'ADNc De façon plus générale, elle peut être utilisée pour éliminer toute séquence indésirable d'une banque Le choix des séquences indésirables peut être fait :
- après analyse d'un échantillon de clones (par exemple 2 000 à 3 000 clones),
- délibérément pour éliminer d'office certaines séquences contaminant classiquement les banques d'ADNc (séquences ribosomiques ou mitochondriales).

En particulier, le procédé selon la présente invention permet d'éliminer les clones les plus redondants, par exemple les TOP20, c'est-à-dire les clones correspondant aux 20 séquences les plus abondantes, de chaque banque. Les calculs réalisés ont montré que l'élimination des TOP20 dans une banque d'ADNc permettait d'augmenter de façon considérable la représentation relative des clones restants

Dans ce mode de réalisation, on pourra par exemple adapter les rapports molaires entre chaque oligonucléotide employé et sa séquence cible, afin d'éliminer simultanément et de façon optimale des séquences différentes

Il est aussi possible de mettre en oeuvre le procédé selon l'invention, soit pour normaliser la distribution de séquences dans une banque, soit pour éliminer en aveugle des clones

Dans le cas de la normalisation, le protocole appliqué pourrait être le suivant :
1) réaliser une préparation plasmidique (double brin) de la banque,
2) à partir de la préparation plasmidique, synthétiser des fragments d'ADN ou de PNA simple brin couvrant toutes les insertions, ou une partie d'entre elles,
3) utiliser les fragments d'ADN simple brin comme matrice pour la formation de triplex catalysés par RecA ou former directement les triplex avec lesdits fragments de PNA,
4) méthyler les séquences non engagées dans des triplex,
5) casser les triplex,
6) digérer les préparations plasmidiques avec une ou deux enzymes de restriction (enzymes dont le site de restriction est à 4 bases) sensibles à la méthylation,
7) digérer les ADNs linéarisés à l'exonucléase,
8) cloner et propager les plasmides intacts

Dans le cas de l'élimination en aveugle de clones, la manipulation consisterait à
1) ordonnancer 2 000 à 4 000 clones,
2) regrouper ces clones pour produire à partir de ces clones des sondes ADN simple brin,
3) utiliser les ADNs simple brin pour éliminer les séquences correspondantes de la banque d'origine,
4) par la suite, de 2 à 4 000 nouveaux clones peuvent être ordonnancés, séquencés et de même utilisés pour éliminer les séquences correspondantes de la banque résiduelle.

La présente invention concerne enfin les banques d'ADNc ainsi obtenues par la mise en oeuvre du procédé décrit précédemment.

Les exemples ci-après permettront de mettre en évidence d'autres caractéristiques et avantages de la présente invention
La figure 1 schématise le procédé selon la présente invention
La figure 2 présente les résultats obtenus sur gel d'agarose, pour la linéarisation d'un plasmide circulaire par digestion ciblée sur un site de restnction, en utilisant le procédé selon l'invention.
La figure 3 présente les résultats d'élimination d'un plasmide ciblé au sein d'un mélange de plasmides, en utilisant le procédé selon l'invention.
La figure 4 présente les séquences des insertions de deux plasmides ciblés CL1 et CL13 Les oligonucléotides utilisés pour mettre en oeuvre le procédé d'élimination selon l'invention, respectivement, OCL1 et OCL13, sont soulignés ; les sites de restriction utilisés, respectivement MspI et HaeIII sont représentés en caractère gras

### Stratégie de protection et d'élimination par RecA

Cette figure résume la procédure utilisée pour éliminer des séquences spécifiques d'une banque d'ADNc en utilisant la protéine RecA Elle montre les effets du protocole sur le plasmide d'un des clones ciblés parmi les TOP20 (plasmide A), et sur le plasmide d'un clone non ciblé (plasmide B) Après formation du filament présynaptique, le complexe oligonucléotide-RecA est mélangé à la préparation d'ADN double brin plasmidique Puisque l'oligonucléotide est choisi spécifiquement pour hybrider à celles des 20 séquences les plus abondantes correspondant à l'insertion du plasmide A, celui-ci peut former un triplex avec l'oligonucléotide complexé avec la protéine, contrairement au plasmide B. La méthylation modifie tous les sites de l'enzyme de restriction choisie (régions hachurées) sur les plasmides A et B, à l'exception du site du plasmide A protégé par la formation du triplex. Après dénaturation de la protéine RecA, l'addition de l'enzyme de restriction génère la linéarisation du plasmide A, alors que le plasmide B reste circulaire. L'étape finale est une hydrolyse du plasmide A (linéarisé) par l'exonucléase. Ceci rend ce plasmide linéarisé plus court, et sûrement non clonable Cette étape n'affectera pas la plasmide-circulaire B.

### Matériels et méthodes

Durant la procédure expérimentale, le matériel suivant est utilisé:
BRM (Buffer RecA and Methylase) . 250 mM tris acétate, 40 mM acétate de magnésium.
RecA protéine : Promega. N° M1692. A 2,58 mg/ml
ATP(γS) Adenosine-5'-O-(3-thiotriphosphate), 10 mM Boehringer Manheim N° 83317521-12
BSA . Bovine Serum Albumine, 10mg/ml New England Biolabs Fournie avec les enzymes
DTT. 100 mM
Acétate de magnésium 80 mM
HaeIII méthylase. New England Biolabs N° 224L. 10,000 U/ml
MspI méthylase New England Biolabs N° 215L 5,000 U/ml
SAM S-Adénosylméthionine 32mM. New England Biolabs Fournie avec les enzymes
Enzyme de restriction HaeIII New England Biolabs N° 108L 10,000-U/ml
Enzyme de restriction MspI New England Biolabs. N° 106L 20,000 U/ml
Exonucléase III. New England Biolabs N° 206L 100,000 U/ml.
Ampicilline Sigma N° A-9518
X-gal Sigma N° B-9146
ITPG Sigma N° I-6758.
(COLONY PICKER Hybaid)

Les différents protocoles utilisés durant la procédure sont les suivants :

### Protocole 1 : Formation du filament présynaptique

Dans un tube Eppendorf de 1,5 ml ajouter 1 µl de BRM, 6,25 µg de la protéine RecA, 160 ng de l'oligonucléotide, et compléter à 9 µl avec de l'eau distillée Mélanger doucement en pipettant, et incuber une minute à 37°C Ajouter 1 µl ATP(γS) (gardé à - 80°C), et incuber 10 minutes à 37°C. L'oligonucléotide et la protéine RecA sont mélangés dans un rapport molaire de 3 l, tenant compte du fait qu'un monomère de la protéine se lie sur la séquence nucléotidique toutes les 3 bases

### Protocole 2 Formation du triplex

Dans un tube Eppendorf de 1,5 ml mélanger doucement 1µg d'ADN plasmidique, 2 µl de BRM, 2,7 µl de DTT, 3 µl de BSA, et compléter à 17 µl avec de l'eau distillée Ajouter cette mixture à la solution précédente, et incuber 40 minutes à 37°C

### Protocole 3 Méthylation

A la solution précédente, ajouter 20 unités de méthylase, de l'acétate de magnésium et du SAM à des concentrations finales respectives de 8 mM et 80 mM, et compléter à 40 µl avec de l'eau distillée. Incuber 45 minutes à 37°C A la fin de la réaction, chauffer la solution 15 minutes à 65°C pour arrêter la méthylation et dénaturer le complexe oligonucléotide-RecA La solution est ensuite purifiée par une extraction phénolique suivie d'une précipitation à l'acétate de sodium 0,3 M dans l'éthanol

### Protocole 4 Digestion par l'enzyme de restriction

Reprendre le culot dans 10 µl d'eau distillée. Ajouter 2,5 µl du tampon 10X de l'enzyme, 20 unités de l'enzyme de restriction, compléter à 25 µl avec de l'eau distillée et incuber une heure à 37°C Finalement, la solution est purifiée par une extraction phénolique suivie d'une précipitation à l'acétate de sodium 0,3 M dans l'éthanol

### Protocole 5 digestion par l'exonucléase III

Reprendre le culot dans 10 µl d'eau distillée. Ajouter 5 µl du tampon 10X de l'enzyme, 400 unités de l'exonucléase, et compléter à 50 µl avec de l'eau distillée Incuber 45 minutes à 37°C Purifier l'ADN par une extraction phénolique, et précipiter à l'éthanol en présence de l'acétate de sodium 0,3 M. Reprendre le culot dans 10 µl d'eau distillée

L'ADN ainsi obtenu est utilisé pour transformer des bactéries compétentes

### Résultats

### Linéarisation d'un plasmide circulaire par digestion ciblée sur un site de restriction

### Exemple I : Efficacité de l'utilisation de la protéine RecA

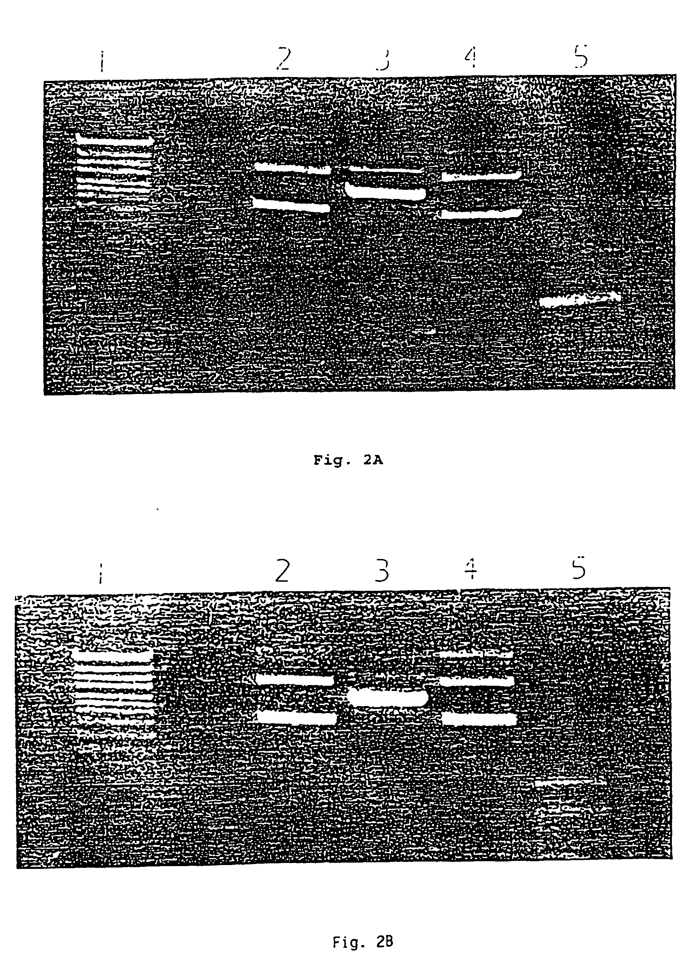

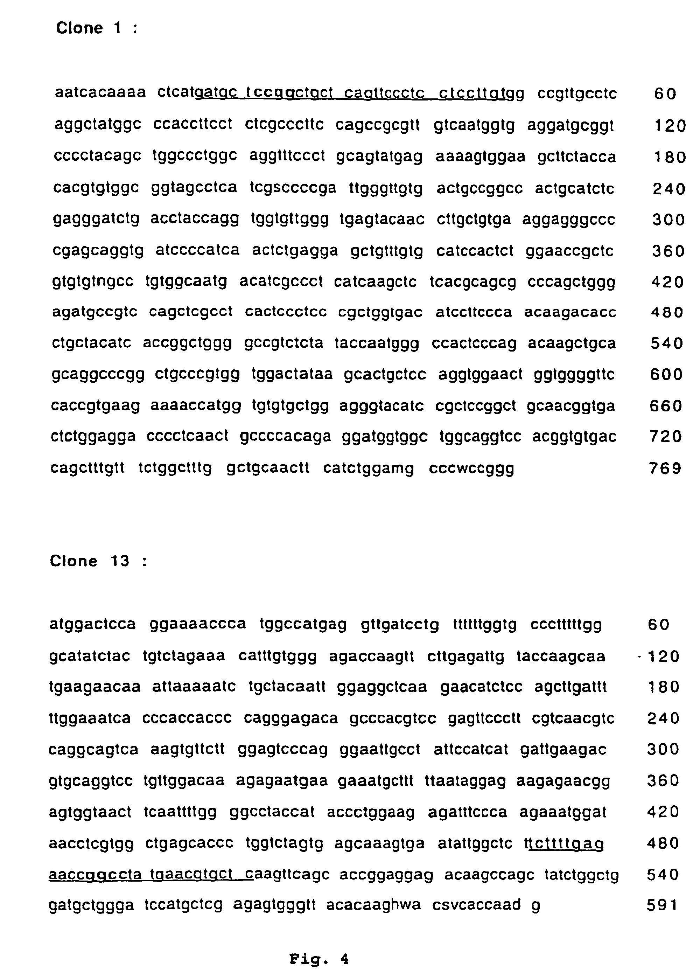

Cette expérience est effectuée sur deux clones d'ADNc (CL1 et CL13) d'abondances différentes d'une banque de pancréas. L'un des deux clones (CL1) correspond à un ADNc dont l'abondance est de 15 %, la séquence correspondant à l'autre clone (CL13) est présente à 1 % dans la banque Les oligonucléotides OCL1 et OCL13 ont été choisis dans les conditions décrites ci-dessus , ils contiennent respectivement les sites de restriction MspI (C*CGG) et HaeIII (GG*CC), aux positions 16 et 473 respectivement sur les séquences des clones correspondants , les séquences ciblées et les oligonucléotides OCL1 et OCL13 sont représentés figure 4. La protéine RecA est complexée aux oligonucléotides OCL1 et OCL13. Les filaments présynaptiques obtenus sont ensuite mélangés aux préparations de plasmides provenant des clones CL1 et CL13 respectivement. Les résultats sur gel d'agarose à 0,8 % (figure 2) montrent que pour les produits de la procédure d'élimination, plasmides protégés par RecA, méthylés, puis digérés par une enzyme de restriction (pistes 3), l'ADN plasmidique a été linéarisé, et montre un profil de migration correspondant à une bande unique de 3 Kb qui migre entre celle de l'ADN superenroulé et celle de l'ADN circulaire Les profils de migration des plasmides non protégés, puis méthylés et digérés par une enzyme de restriction (pistes 4) et des plasmides natifs (pistes 2) sont similaires, ce qui montre que les conditions de méthylation sont suffisantes pour modifier tous les sites de restriction non protégés ; aucune digestion n'a lieu. La multitude de bandes qu'on voit pour les plasmides non protégés, non méthylés, mais digérés par les enzymes de restriction (pistes 5) est due au fait que les sites des enzymes MspI et HaeIII sont présents respectivement en 13 et 14 copies dans le vecteur de clonage (BlueScript II SK(-), Stratagene, N° 212206) Ceci entraîne la coupure du vecteur en plusieurs sites

### Elimination spécifique d'un plasmide ciblé au sein d'un mélange de plasmides

### Exemple II : Efficacité de l'utilisation de la protéine RecA : deux systèmes modèles différents

Pour tester l'efficacité de l'élimination d'un clone abondant en utilisant la protéine RecA, deux expériences ont été faites.

Dans la première expérience, l'ADN double brin traité est constitué de l'une de nos deux préparations précédentes de plasmides (CL1 et CL13), mélangée à une préparation de plasmides correspondant à un vecteur de clonage (pBSSK). Les deux clones 1 et 13 ont été séparément mélangés avec le plasmide pBSSK dans une proportion équivalente à leur abondance dans la banque de pancréas, soit 15 % et 1 % respectivement. L'élimination selon l'invention utilisant la protéine RecA a été réalisée en employant les oligonucléotides OCL1 et OCL13, suivant le protocole décrit précédemment Le produit final a été utilisé pour transformer des bactéries électrocompétentes (Epicurian Coli SURE, Stratagene N° 2000227) et la solution obtenue étalée sur des boîtes de LB agar avec ampicilline, X-gal, et ITPG Le nombre de clones recombinants (colonies blanches contenant des insertions correspondant aux séquences des clones 1 ou 13) a été compté avant et après traitement d'élimination. Pour le clone 1, le pourcentage de clones recombinants est de 9 % avant traitement et 1,4 % après l'élimination de ce clone Pour le clone 13, la chute est de 1,5 % à 0,28 % Ces deux tests montrent une efficacité d'élimination de 84 %.

Dans la deuxième expérience, une préparation de plasmides correspondant à la totalité de la banque de pancréas a constitué l'ADN double brin traité par la procédure d'élimination. Celle-ci a été effectuée avec l'oligonucléotide OCL1, dans le but d'éliminer de la banque totale le clone CL1

La banque d'ADNc de pancréas humain est une banque correspondant aux extrémités 5' des ARNm de pancréas Ainsi, comme l'oligonucléotide OCL1 est choisi à proximité de l'extrémité 5' (position 16), tous les clones correspondant à l'extrémité 5' de ce messager devraient être éliminés Dans une banque d'ADNc amorcée dT, la même manipulation peut être réalisée sur l'extrémité 3' des ARNs messagers Par contre, dans le cas d'une banque amorcée aléatoirement, le choix des oligonucléotides est plus délicat Ceci peut être contourné soit en trouvant, grâce à des logiciels informatiques, des homologies entre les séquences et en choisissant les oligonucléotides dans la région conservée, soit en synthétisant des sondes ADN simple brin à partir des insertions en utilisant des séquences du site de clonage multiple comme amorces.

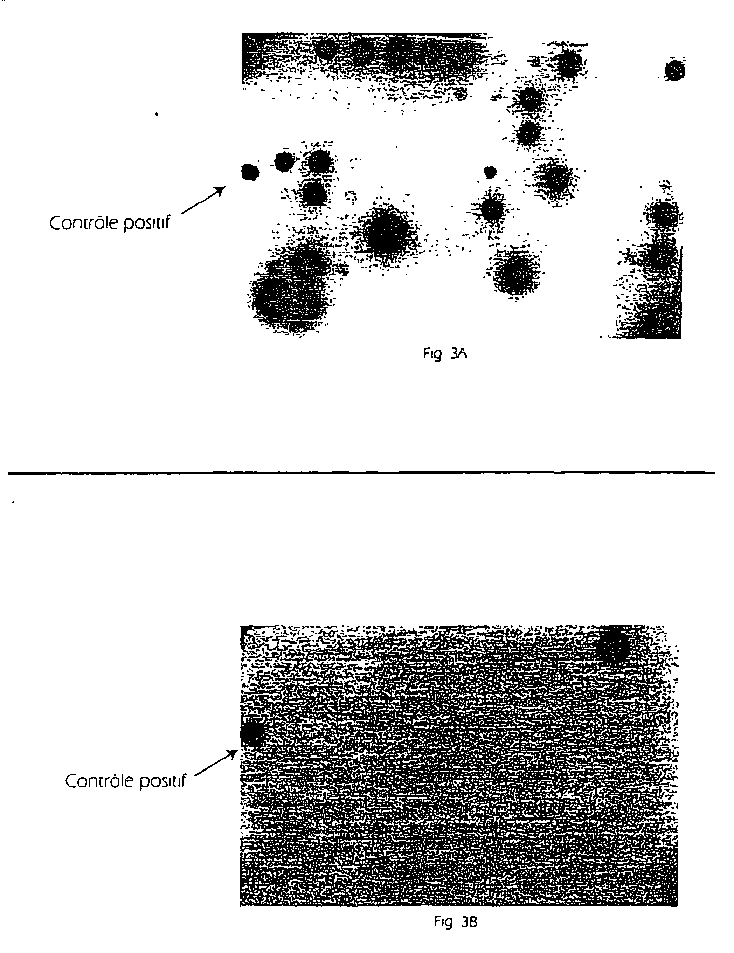

Après formation du filament présynaptique avec l'oligonucléotide OCL1 et protection, la méthylation a été faite avec la MspI méthyltransférase Ensuite l'enzyme MspI a été ajoutée et le mélange de digestion traité à l'exonucléase III Des bactéries électrocompétentes ont été transformées par le produit final et étalées sur des boîtes de LB agar En théorie, tous les plasmides de la banque, hormis ceux portant la séquence correspondant à l'insertion du clone 1, vont être complètement méthylés, et donc non digérés par l'enzyme MspI. Des ACPs (Amplification en Chaîne par Polymérase) sur 96 colonies recombinantes ont été effectuées, utilisant comme amorces d'amplification les amorces "reverse" et "universelle" classiques, pour sortir les insertions des plasmides. Les produits d'ACP ont été spottés sur des filtres en nylon chargés positivement (Amersham Life Sciences), et les filtres ainsi obtenus hybridés en utilisant l'oligonucléotide phosphorylé au P32 (OCL1) spécifique du clone d'intérêt (CL1). Les résultats de cette expérience (figure 3) montrent une efficacité d'élimination de 85 % : le nombre de signaux positifs chute de 21 avant le traitement à 3 après élimination du clone en question.

### Références

Adams et al (1995) "Initial assessment of human gene diversity and expression patterns based upon 83 million nucleotides of cDNA sequence" Nature, 377 supp, 3-174
Britten R.J, Grahan D.E , Neufeld B.R (1974) "Analysis of repeating DNA sequences by reassociation" Method in Enzymol, 29, 363-418
Davidson E H et Britten R J (1979) "Regulation of Gene Expression : Possible Role of Repetitive Sequences" Science 204, 1052-1059
Koob M , Barbiewicz A, Ku J , Szybalski W (1992) "RecA-AC single site cleavage of plamids and chromosomes at any predetermined restriction site" Nucleic Acids Res., 21, 5831-5836.
Lanfranchi et al. (1985) "A new method for the construction of 3'-end specific cDNA libraries and its application for the identification of 4370 expressed sequence tags (ESTs) from human skeletal muscle" Genome Science & Technology, 1, 63.
Patanjali S.R, Parimoo S et Weissman S M (1991) "Construction of a uniform abundance (normalized) cDNA library" PNAS 8, 1943-1947.
Sasaki Y.F, Ayusawa D. et Orshi M (1994) "Construction of a normalized cDNA library by introduction of a semi-solid RNA-cDNA hybridization system" NAR 22, 907-922
Soares M B et Efstratiadis A (1995) "Method for construction of normalized cDNA library" PCT-US 94/10821.
Soares M.B , Fatima Bonaldo F , Jelene P , Su L , Lawton L et Efstratiadis A (1994) "Construction and characterization for normalized cDNA library" PNAS 91, 9228-9232.
Veselkov A.G., Demidov V.V., Frank-Kamenetskii M.D , Nielsen P C (1996) "PNA as a rare genome-cutter", Nature 379, 214.
Weisman S.M (1987) "Molecular genetic techniques for mapping the human genome" Mol Biol Med, 4, 133-143.
Young B.D. et Anderson ML M (1985) in Nucleic acid hybridization, a practical approach (Yames B.D et Higgins S.J. ed), 47-72.

## Revendications

1. Procédé destiné à éliminer spécifiquement au moins un type de plasmide d'ADN double brin dans un ensemble de plasmides, **caractérisé en ce que** :
- on prépare des fragments d'acides nucléiques capables de former des structures en triplex et dont la séquence correspond à une séquence du plasmide à éliminer et comporte, en outre, un site de reconnaissance pour une enzyme de restriction sensible à la méthylation ;
- on mélange l'ensemble des plasmides avec lesdits fragments d'acides nucléiques pour former des triplex sur les plasmides à éliminer ;
- on méthyle le mélange d'ADN obtenu au moyen de méthyltransférase spécifique des sites de reconnaissance de l'enzyme de restriction sensible à la méthylation utilisée à l'étape suivante ;
- on libère les triplex ;
- on digère le mélange obtenu avec l'enzyme de restriction sensible à la méthylation ;
- si nécessaire, on élimine ou on sépare les ADNs double brin linéaires correspondant aux plasmides à éliminer.

2. Procédé destiné à éliminer spécifiquement au moins un type de plasmide d'ADN double brin d'un ensemble de plasmides, **caractérisé en ce que** :
- on prépare des filaments présynaptiques par fixation d'une protéine à activité RecA sur un ADN simple brin dont la séquence correspond à une séquence du plasmide à éliminer et comporte, en outre, un site de reconnaissance pour une enzyme de restriction sensible à la méthylation ;
- on mélange l'ensemble des plasmides avec les filaments présynaptiques pour former des triplex sur les plasmides à éliminer ;
- on méthyle le mélange d'ADN obtenu au moyen de méthyltransférase spécifique des sites de reconnaissance de l'enzyme de restriction sensible à la méthylation utilisée à l'étape suivante ;
- on libère les triplex ;
- on digère le mélange obtenu avec l'enzyme de restriction sensible à la méthylation ;
- si nécessaire, on élimine ou on sépare les ADNs double brin linéaires correspondant aux plasmides à éliminer.

3. Procédé selon la revendication 1, **caractérisé en ce que** l'on forme des structures en triplex par hybridation des séquences cibles avec des PNAs.

4. Procédé selon l'une des revendications 1 à 3, **caractérisé en ce que** l'ensemble de plasmides de départ est une banque d'ADN.

5. Procédé selon l'une des revendications 1 à 3, **caractérisé en ce que** l'ensemble de plasmides de départ est une banque d'ADNc.

6. Procédé selon l'une des revendications 1 à 5, **caractérisé en ce qu'**on souhaite éliminer une séquence spécifique de la banque.

7. Procédé selon l'une des revendications 1 à 5, **caractérisé en ce qu'**on souhaite éliminer plusieurs séquences spécifiques.

8. Procédé selon l'une des revendications 1 à 7, **caractérisé en ce qu'**on souhaite éliminer spécifiquement les plasmides portant une séquence parmi les plus abondantes.

9. Procédé selon l'une des revendications 1 à 7, **caractérisé en ce qu'**on souhaite éliminer spécifiquement les plasmides portant plusieurs séquences parmi les plus abondantes.

10. Procédé selon l'une des revendications 1 à 7, **caractérisé en ce qu'**on souhaite éliminer spécifiquement les plasmides portant les séquences les plus abondantes.

11. Procédé selon l'une des revendications 1 à 10, **caractérisé en ce que** la protéine à activité RecA est la protéine RecA.

12. Procédé selon l'une des revendications 1 à 11, **caractérisé en ce que** le triplex est formé en présence de cations divalents.

13. Procédé selon la revendication 12, **caractérisé en ce que** le triplex est formé en présence d'ATP(γS).

14. Procédé selon l'une des revendications 1 à 13, **caractérisé en ce que** la méthylation est effectuée par des méthyltransférases.

15. Procédé selon l'une des revendications 1 à 14, **caractérisé en ce que** l'enzyme de restriction sensible à la méthylation est choisie parmi HaeIII et MspI.

16. Procédé selon l'une des revendications 1 à 15, **caractérisé en ce que** les ADNs double brin linéaires sont éliminés par hydrolyse.

17. Procédé selon la revendication 16, **caractérisé en ce que** l'hydrolyse est effectuée par une exonucléase.

18. Banque d'ADNc obtenue par la mise en oeuvre du procédé selon l'une des revendications 1 à 17, **caractérisée en ce qu'**elle est normalisée au moyen d'une enzyme de restriction sensible à la méthylation et par soustraction d'au moins une séquence spécifique d'une espèce moléculaire, **caractérisée en ce qu'**elle comporte au moins un ADNc présentant au moins un groupement méthyle fixé lors de l'étape de méthylation du mélange d'ADN et **caractérisée en ce que** lesdits ADNc ainsi obtenus sont capables de transformer des cellules de bactérie.

## Patentansprüche

1. Verfahren, welches dazu bestimmt ist, spezifisch wenigstens eine Art von doppelstrangigem DNA-Plasmid in einer Gesamtheit von Plasmiden zu eliminieren, **dadurch gekennzeichnet, dass**:
- man Nukleinsaurefragmente herstellt, welche in der Lage sind, dreifachstrangige Strukturen (Triplex-Strukturen) zu bilden, und deren Sequenz einer Sequenz des zu eliminierenden Plasmids entspricht und außerdem eine Erkennungsstelle fur ein gegenuber Methylierung empfindliches Restriktionsenzym umfasst;
- man die Gesamtheit der Plasmide mit den Nukleinsaurefragmenten mischt, um Dreifachstrange mit den zu eliminierenden Plasmiden zu bilden;
- man die erhaltene DNA-Mischung methyliert mittels einer fur die Erkennungsstellen des gegenuber Methylierung empfindlichen Restriktionsenzyms, das in dem folgenden Schritt eingesetzt wird, spezifischen Methyltransferase;
- man die Dreifachstrange freigibt (dissoziieren lasst);
- man die erhaltene Mischung mit dem gegenuber Methylierung empfindlichen Restriktionsenzym verdaut;
- sofern erforderlich, man die linearen doppelstrangigen DNAs, welche den zu eliminierenden Plasmiden entsprechen, eliminiert oder abtrennt.

2. Verfahren, welches dazu bestimmt ist, spezifisch wenigstens eine Art von doppelstrangigem DNA-Plasmid aus einer Gesamtheit von Plasmiden zu eliminieren, **dadurch gekennzeichnet, dass**:
- man prasynaptische Filamente herstellt durch Anheftung eines Proteins mit RecA-Aktivitat an eine einzelstrangige DNA, deren Sequenz einer Sequenz des zu eliminierenden Plasmids entspricht und außerdem eine Erkennungsstelle fur ein gegenuber Methylierung empfindliches Restriktionsenzym umfasst;
- man die Gesamtheit der Plasmide mit den prasynaptischen Filamenten mischt, um Dreifachstrange (Triplex-Strange) mit den zu eliminierenden Plasmiden zu bilden;
- man die erhaltene DNA-Mischung methyliert mittels einer fur die Erkennungsstellen des gegenuber Methylierung empfindlichen Restriktionsenzyms, das in dem folgenden Schritt eingesetzt wird, spezifischen Methyltransferase;
- man die Dreifachstrange freigibt (dissoziieren lasst);
- man die erhaltene Mischung mit dem gegenuber Methylierung empfindlichen Restriktionsenzym verdaut;
- sofern erforderlich, man die linearen doppelstrangigen DNAs, welche den zu eliminierenden Plasmiden entsprechen, eliminiert oder abtrennt.

3. Verfahren nach Anspruch 1, **dadurch gekennzeichnet, dass** man dreifachstrangige Strukturen (Triplex-Strukturen) durch Hybridisierung der Zielsequenzen mit PNAs bildet.

4. Verfahren nach einem der Anspruche 1 bis 3, **dadurch gekennzeichnet, dass** die Gesamtheit von Ausgangsplasmiden eine DNA-Bank ist.

5. Verfahren nach einem der Anspruche 1 bis 3, **dadurch gekennzeichnet, dass** die Gesamtheit von Ausgangsplasmiden eine cDNA-Bank ist.

6. Verfahren nach einem der Anspruche 1 bis 5, **dadurch gekennzeichnet, dass** man eine spezielle Sequenz aus der Bank zu eliminieren wunscht.

7. Verfahren nach einem der Anspruche 1 bis 5, **dadurch gekennzeichnet, dass** man mehrere spezielle Sequenzen zu eliminieren wunscht.

8. Verfahren nach einem der Anspruche 1 bis 7, **dadurch gekennzeichnet, dass** man spezifisch die Plasmide, welche eine Sequenz unter den am haufigsten vorkommenden aufweisen, zu eliminieren wunscht.

9. Verfahren nach einem der Anspruche 1 bis 7, **dadurch gekennzeichnet, dass** man spezifisch die Plasmide, welche mehrere Sequenzen unter den am haufigsten vorkommenden aufweisen, zu eliminieren wunscht.

10. Verfahren nach einem der Anspruche 1 bis 7, **dadurch gekennzeichnet, dass** man spezifisch die Plasmide, welche die am haufigsten vorkommenden Sequenzen aufweisen, zu eliminieren wunscht.

11. Verfahren nach einem der Anspruche 1 bis 10, **dadurch gekennzeichnet, dass** das Protein mit RecA-Aktivitat das RecA-Protein ist.

12. Verfahren nach einem der Anspruche 1 bis 11, **dadurch gekennzeichnet, dass** der Dreifachstrang in Gegenwart von zweiwertigen Kationen gebildet wird.

13. Verfahren nach Anspruch 12, **dadurch gekennzeichnet, dass** der Dreifachstrang in Gegenwart von ATP(γS) gebildet wird.

14. Verfahren nach einem der Anspruche 1 bis 13, **dadurch gekennzeichnet, dass** die Methylierung durch Methyltransferasen erfolgt.

15. Verfahren nach einem der Anspruche 1 bis 14, **dadurch gekennzeichnet, dass** das gegenuber Methylierung empfindliche Restriktionsenzym unter HaeIII und MspI ausgewahlt wird.

16. Verfahren nach einem der Anspruche 1 bis 15, **dadurch gekennzeichnet, dass** die linearen doppelstrangigen DNAs durch Hydrolyse eliminiert werden.

17. Verfahren nach Anspruch 16, **dadurch gekennzeichnet, dass** die Hydrolyse durch eine Exonuklease erfolgt.

18. cDNA-Bank, welche durch das Ausfuhren des Verfahrens nach einem der Anspruche 1 bis 17 erhalten wird, **dadurch gekennzeichnet, dass** sie mittels eines gegenuber Methylierung empfindlichen Restriktionsenzyms und durch Entfernung wenigstens einer fur eine Molekulspezies spezifischen Sequenz normalisiert ist, **dadurch gekennzeichnet, dass** sie wenigstens eine cDNA umfasst, welche wenigstens eine Methylgruppe, die wahrend des Methylierungsschritts der DNA-Mischung angeheftet worden ist, aufweist, und **dadurch gekennzeichnet, dass** die so erhaltenen cDNAs in der Lage sind, Bakterienzellen zu transformieren.

## Claims

1. Method for specifically eliminating at least one type of double-strand DNA plasmid from a set of plasmids, **characterized in that**:
- fragments of nucleic acids are prepared which are capable of forming triplex structures and whose sequence corresponds to a sequence of the plasmid to be eliminated and which further comprises a recognition site for a restriction enzyme sensitive to methylation;
- the set of plasmids is mixed with said fragments of nucleic acids to form triplexes on the plasmids to be eliminated;
- the DNA mixture obtained is methylated by means of methyltransferase specific for the recognition sites for the restriction enzyme sensitive to methylation used in the next step;
- the triplexes are released;
- the mixture obtained is digested with the methylation-sensitive restriction enzyme;
- if necessary, the linear double-strand DNAs corresponding to the plasmids to be eliminated are eliminated or separated.

2. Method for specifically eliminating at least one type of double-strand DNA plasmid from a set of plasmids, **characterized in that**:
- presynaptic filaments are prepared by binding a protein with RecA activity to a single-strand DNA whose sequence corresponds to a sequence of the plasmid to be eliminated and which further comprises a recognition site for a restriction enzyme sensitive to methylation;
- the set of plasmids is mixed with the presynaptic filaments to form triplexes on the plasmids to be eliminated;
- the DNA mixture obtained is methylated by means of methyltransferase specific for the recognition sites for the restriction enzyme sensitive to methylation used in the next step;
- the triplexes are released;
- the mixture obtained is digested with the methylation-sensitive restriction enzyme;
- if necessary, the linear double-strand DNAs corresponding to the plasmids to be eliminated are eliminated or separated.

3. Method according to Claim 1, **characterized in that** triplex structures are formed by hybridization of the target sequences with PNAs.

4. Method according to one of Claims 1 to 3, **characterized in that** the set of starting plasmids is a DNA library.

5. Method according to one of Claims 1 to 3, **characterized in that** the set of starting plasmids is a cDNA library.

6. Method according to one of Claims 1 to 5, **characterized in that** it is desired to eliminate a specific sequence from the library.

7. Method according to one of Claims 1 to 5, **characterized in that** it is desired to eliminate several specific sequences.

8. Method according to one of Claims 1 to 7, **characterized in that** it is desired to specifically eliminate the plasmids carrying a sequence which is among the most abundant.

9. Method according to one of Claims 1 to 7, **characterized in that** it is desired to specifically eliminate the plasmids carrying several sequences which are among the most abundant.

10. Method according to one of Claims 1 to 7, **characterized in that** it is desired to specifically eliminate the plasmids carrying the most abundant sequences.

11. Method according to one of Claims 1 to 10, **characterized in that** the protein with RecA activity is the RecA protein.

12. Method according to one of Claims 1 to 11, **characterized in that** the triplex is formed in the presence of divalent cations.

13. Method according to Claim 12, **characterized in that** the triplex is formed in the presence of (γS) ATP.

14. Method according to one of Claims 1 to 13, **characterized in that** the methylation is carried out by methyltransferases.

15. Method according to one of Claims 1 to 14, **characterized in that** the restriction enzyme sensitive to methylation is chosen from HaeIII and MspI.

16. Method according to one of Claims 1 to 15, **characterized in that** the linear double-strand DNAs are eliminated by hydrolysis.

17. Method according to Claim 16, **characterized in that** the hydrolysis is carried out by an exonuclease.

18. cDNA library obtained using the method according to one of Claims 1 to 17, **characterized in that** it is normalized by means of a restriction enzyme sensitive to methylation and by subtraction of at least one specific sequence from a molecular species, **characterized in that** it comprises at least one cDNA having at least one methyl group bound during the step of methylation of the DNA mixture and **characterized in that** the said cDNAs thus obtained are capable of transforming bacterial cells.
